# EUROPÄISCHE PATENTSCHRIFT

(11) **EP 2 373 550 B1**
(45) Veröffentlichungstag und Bekanntmachung des Hinweises auf die Patenterteilung: **10.02.2016**
(21) Anmeldenummer: 09756434.8
(22) Anmeldetag: 17.11.2009
(51) Int. Cl.: B65D 47/24, F16K 3/26

(54) **VERSCHLUSS FÜR EINEN BEHÄLTER**
CLOSURE FOR A CONTAINER
BOUCHON POUR UN CONTENANT

(30) Priorität: 05.12.2008 DE 102008060773
(43) Veröffentlichungstag der Anmeldung: 12.10.2011
(73) Patentinhaber: Sartorius Stedim Biotech GmbH, 37079 Göttingen (DE)
(72) Erfinder: REIF, Oscar-Werner, 30173 Hannover (DE); FATHERAZI, Shahmir, 52066 Aachen (DE); GRELLER, Gerhard, 37085 Göttingen (DE); BATES, Michael, GL3 4PD (GB); BARBAROUX, Magali, F-13112 La Destrousse (FR)
(74) Vertreter: Müller-Boré & Partner Patentanwälte PartG mbB
(86) Internationale Anmeldenummer: PCT/EP2009/008176
(87) Internationale Veröffentlichungsnummer: WO 2010/063372

(56) Entgegenhaltungen:
- EP-A1- 1 065 150
- US-A1- 2004 129 741
- US-B1- 7 588 142

## Beschreibung

Die Erfindung betrifft einen Verschluss für einen Behälter, welcher eine Zu- und Abführung von Medien gewährleistet. Ein derartiger Verschluss wird insbesondere für Einweg-, Misch- und Bioreaktoren in der Pharmazie und Biotechnologie eingesetzt.

Der Verschluss eines Behälters ist in vielen verschiedenen Ausführungsformen bekannt. Für Behälter im Bereich der Labortechnologie, im industriellen Gebrauch, wie der pharmazeutischen Produktherstellung oder der Lebensmitteltechnologie, werden Verschlüsse benötigt, die einfach und sicher zu handhaben sind. Insbesondere im Bereich der Einwegprodukte ist eine besonders kostengünstige Herstellung nötig.

Die Druckschrift US 2004/0129741 A1 offenbart einen Verschluß für Trinkflaschen, welcher ein Basiselement umfaßt, das über ein Gewinde mit einem Flaschenkörper verbindbar ist. Weiterhin umfaßt der Verschluß einen Ausgießer, welcher auf dem Basiselement hin- und herbewegt werden kann, um die Flasche zu öffnen oder zu verschließen.

Die Druckschrift EP 1 065 150 A1 offenbart einen antiseptischen Verschluß für Behälter, welcher einen Hauptkörper sowie einen oberen Verschluß umfaßt. Der Hauptkörper ist mit einem Behälter verbindbar. Der obere Verschluß ist axial beweglich mit dem Hauptkörper verbunden, wobei durch ein Hin- und Herbewegen des oberen Verschlußes der antiseptische Verschluß geöffnet bzw. geschlossen werden kann.

Aus US 5,975,369 ist ein Trinkflaschenverschluss bekannt, bei welchem eine mit der Trinkflasche verbundene, erste Hülse einen zylindrischen Stempel aufweist. Der Verschluss weist weiterhin eine zweite zylindrische Hülse mit einem kleineren Durchmesser als dem Durchmesser der mit der Flasche verbundenen, ersten Hülse auf, wobei die zylindrische Hülse mit kleinerem Durchmesser an ihrem oberen Ende eine Öffnung aufweist, welche in geschlossenem Zustand der Flasche durch den zylindrischen Stempel verschlossen ist. Die zylindrische Hülse mit kleinerem Durchmesser ist entlang des Stempels axial verschiebbar. Durch Arretierungseinrichtungen am Stempel und der Hülse mit dem kleineren Durchmesser (z. B. durch Anschläge an der Innenwandung der Hülse mit kleinerem Durchmesser) wird ein Abziehen der Hülse mit dem kleineren Durchmesser von dem Stempel ausgeschlossen. Hierbei erweist sich als nachteilig, dass ein Totraum im Verschluss selbst existiert. Konstruktionsbedingt sind zwischen den zylindrischen Hülsen und am Stempel Freiräume. Der bestimmungsgemäße Gebrauch für beispielsweise Trinkflaschen lässt einen Rückfluss des Mediums in den Behälter zu, da eine Anbringung an der Behälterdecke vorgesehen ist. Bei Anbringung am Boden eines Behälters würde ein solcher Verschluss Platz für ein Medium schaffen, welches in diesem Freiraum verweilt. Für die Verwendung an einem Einweg-, Misch- oder Bioreaktor ist ein solcher Verschluss ungünstig, da der im Totraum des Verschlusses verbleibende Anteil des Mediums nicht den gleichen Konditionen unterliegt wie das Medium im Behälter. Auch können Partikel in den Totraum sedimentieren und diesen verstopfen.

US 2003/0121879 A1, US 6,758,359 B2, US 6,321,924 B1 und WO 2005/044685 A1 offenbaren Flaschenverschlüsse für Getränke- und Reinigungsmittelbehälter, die weitere Entwicklungen des in US 5,975,369 offenbarten Verschlusses bezüglich der Ausgestaltung der Hülsen, des Stempels und einer diese Komponenten abdeckenden Schutzkappe darstellen. Auch die Weiterentwicklungen lösen nicht das Problem des Totraumes, da der bestimmungsgemäße Gebrauch gänzlich anders ist.

Schließlich ist aus US 2007/0102450 A1 ein Verschluss für einen Behälter bekannt, der ein Verschlussteil, einen Flansch und zwei Dichtringe aufweist, wobei das Verschlussteil ein Hohlkolben ist, der an einem Ende verschlossen ist und an dem anderen Ende geöffnet ist, wobei unterhalb der verschlossenen Seite Bohrungen vorhanden sind, durch welche das Medium bei geöffnetem Verschluss fließt. Über und unter den Bohrungen befinden sich Dichtungen. Die verschlossene Seite verschließt im geschlossenen Zustand zusammen mit dem oberen Dichtring die Flanschöffnung.

Bei einer ersten, in US 2007/0102450 A1 offenbarten Ausführungsform ist der Hohlkolben zum Öffnen in den Behälter hinein verschiebbar. Nachteilig dabei ist, dass ein in den Behälter hineinragendes Teil störend wirkt gegenüber behälterinnenseitig anzubringenden Elementen, wie zum Beispiel Rührern oder Begasungsvorrichtungen, die bevorzugt, ebenso wie ein Verschluss, mittig angebracht werden.

In einer zweiten Ausführungsform lässt sich der Hohlkolben aus dem Behälter heraus in einen "Vorraum" zurückziehen. Das Medium gelangt bei geöffnetem Zustand des Verschlusses in den Vorraum und von dort aus durch die Bohrungen in den Hohlkolben. Nachteilig ist hier die konstruktionsbedingt fehlende Arretierung, wodurch keine Sicherheit gegen ein unbeabsichtigtes, vollständiges Herausziehen des Hohlkolbens gegeben ist. Ein weiterer Nachteil ist die ungünstige Umlenkung des Behälterinhaltes beim Abflussvorgang. Das Medium wird über das geschlossene Ende und durch die bei geöffnetem Zustand sich im Vorraum befindlichen Bohrungen mehrfach umgelenkt und erfährt somit einen höheren Fließwiderstand. Dies hat einerseits einen negativen Einfluss auf scherempfindliche Substanzen und andererseits verringert sich somit der Volumenstrom, was zu einer längeren Entleerungszeit des Behälters führt. Der Vorraum erweist sich weiterhin als nachteilig, wenn sich der Verschluss in einem geschlossenen Zustand befindet. Im Falle einer Leckage am zum Behälterinneren angebrachten Dichtring, aber auch bei unzureichendem oder fehlerhaftem Verschließen, würde sich der Vorraum mit dem Medium füllen. Bei kleinen Leckagen füllt sich vorerst der Vorraum und das Medium gelangt erst nach einiger Zeit durch die Bohrungen in den Hohlkolben und von dort aus in den Abflussschlauch. Des weiteren entsteht durch den Vorraum ein Totraum, welcher im Hinblick auf hygienisches Design nicht erwünschenswert ist. Somit gestaltet sich eine etwaige Reinigung als problematisch und es könnten sich beispielsweise Bakterien vermehren oder Zellen absterben. Es ist auch möglich, dass zurückbleibende Substanzen sich anderweitig entwickeln und bei einer Entnahme des Mediums, welches durch Öffnung des Verschlusses unweigerlich mit im Vorraum zurückbleibenden Substanzen in Verbindung gerät, entsprechende Analysen verfälscht werden.

Ein gemeinsamer Nachteil beider Ausführungsformen der US 2007/0102450 A1 besteht darin, dass im Falle einer Leckage an beiden Dichtungen oder im geöffnetem Zustand bei einer Leckage an der Dichtung unterhalb der Bohrungen der Behälterinhalt ins Freie austritt. Dies kann zu einer gefährlichen Personal- und/oder Umweltkontamination führen und gefährdet darüber hinaus eine zu wahrende Sterilität.

Der Erfindung liegt daher die Aufgabe zugrunde, einen Verschluss für einen Behälter zu schaffen, der keinen Totraum, eine günstige Medienführung und eine hohe Sicherheit aufweist.

Die Aufgabe wird durch eine Vorrichtung gemäß Anspruch 1 gelöst. Der erfindungsgemäße Verschluss für einen Behälter umfasst ein Oberteil und ein Unterteil. Das Oberteil ist mit dem Behälter verbindbar und weist eine Öffnung auf. Weiterhin weist es einen äußeren Hohlkörper und einen zu diesem koaxial angeordneten, inneren Hohlkörper auf, der mit dem äußeren Hohlkörper einen Zwischenraum ausbildet. Die Öffnung wird durch einen Dorn verschlossen. Dieser ist durch mindestens eine Halteeinrichtung koaxial in einem hohlkörperförmigen Unterteil aufgenommen. Der obere Teil des Unterteils ist zum Öffnen der Öffnung in dem Zwischenraum von dem Behälter weg längsverschiebbar, und die Längsverschiebbarkeit des Unterteils ist durch Arretierungseinrichtungen begrenzt. Somit ist gewährleistet, dass zum Öffnen und Schließen keine Gegenstände in den behälterinnenseitigen Bereich hineinbewegt werden. Während die Handhabung wünschenswert einfach ist, verhindern gleichzeitig die Arretierungseinrichtungen ein Abziehen des Unterteils und übernehmen somit eine wichtige Sicherungsfunktion. Hektisches, schnelles oder zu kräftiges Öffnen wird somit abgefangen. Weiterhin besteht der Verschluss im Wesentlichen aus den zwei Teilen Oberteil und Unterteil, die jeweils einfach und kostengünstig gefertigt und zusammengefügt werden können. Ein weiterer wichtiger Vorteil ist die Art der Medienführung. Durch den geradlinigen Übergang vom Behälter in die Öffnung über den Verschluss in eine Leitung fällt ein Vorraum, und somit Totraum, für das Medium weg. Durch die koaxialen Hohlkörper und das darin längsverschiebbar aufgenommene und führbare hohlkörperförmige Unterteil wird das Medium geradlinig, einfach und schonend in den Behälter hinein oder aus diesem heraus geführt. Darüber hinaus kann das Medium konstruktionsbedingt nicht nach Außen gelangen, wodurch Umweltkontamination und Personenschäden vermieden werden.

Gemäß einer bevorzugten Ausführungsform der Erfindung sind die Arretierungseinrichtungen an der Innenwand des Unterteils und an der Außenwand des inneren Hohlkörpers angeordnet. Dies kann im Spritzgießverfahren einfach und kostengünstig realisiert werden. Die Schub- und Zughandhabung kann somit sicher und einfach zum Öffnen und Verschließen des Verschlusses führen. Gleichzeitig wird ein vollständiges Abziehen effektiv verhindert. Der Aufbau des Verschlusses erlaubt zudem, dass zu dessen Herstellung flexibles Material verwendet werden kann, da die geometrische Anordnung von Außen-, Innenzylinder und dem Unterteil mit Dorn zueinander ein Verbiegen, beispielsweise des Innenzylinders durch kräftiges Öffnen und somit Übereinanderbewegen der Arretierungseinrichtungen, verhindert.

In einer besonders bevorzugten Ausführungsform des erfindungsgemäßen Verschlusses schließt im verschlossenem Zustand das obere Ende des Dorns bündig mit dem Oberteil ab. Somit ergeben sich keine hervorstehenden Teile im Behälter, die behälterinnenseitig eine Anbringung von Elementen wie Rührern, Begasungseinheiten etc. beeinträchtigen. Ebenso wie diese Elemente wird auch ein Verschluss bevorzugt am Behälterboden möglichst mittig angeordnet.

In einer weiteren bevorzugten Ausführungsform des Verschlusses ist der Dorn durch einen Dichtring, beispielsweise einen O-Ring, gegenüber der Öffnung des Oberteils abgedichtet. Hierdurch wird ein sicherer und dichter Verschluss gewährleistet. Eine Ausführungsform kann ein Dichtring um den Dorn herum sein. Eine weitere Ausführungsform besteht beispielsweise bei Anbringung des Dichtringes an der Öffnung.

Bei diesen vorgenannten, bevorzugten Ausführungsformen des erfindungsgemäßen Verschlusses sind im geöffneten Zustand durch die Öffnung Medien durch den Zwischenraum zwischen Innenwand des inneren Hohlkörpers und dem Dorn aus dem Behälter abführbar bzw. in den Behälter zuführbar. Diese vorgenannten Ausführungsformen erlauben, Medien dem Behälter insbesondere steril zuzuführen bzw. aus diesem steril abzuführen.

In einer weiteren vorteilhaften Ausgestaltung der Erfindung weist die Innenwand des äußeren Hohlkörpers und die Außenwand des inneren Hohlkörpers je mindestens ein Dichtungselement auf. Somit kann das Medium steril zu- und abfließen und es gibt keinen Totraum für ein Medium, welches aufgrund fehlerhaften Verschlusses oder leckagenbedingt durch die Öffnung austritt oder bereits ausgetreten ist oder sich nach dem Ab- bzw. Zufluss im Verschluss sammeln kann. Auch dadurch ist eine weitere zusätzliche Sicherung vorhanden, die einen Leckagenaustritt in die Umwelt verhindert. Somit werden Umweltkontaminationen vermieden und eine Gefährdung von Personen wird ausgeschlossen. Bei einem bereits befülltem Behälter wird das Medium aufgrund des geringsten Fließwiderstandes am Dorn vorbei direkt in das Unterteil und von dort hinaus in eine Ableitung fließen. Ebenso verhält sich das Medium bei einer Fließrichtung in den Behälter hinein. Bei hohen Volumenströmen ist es jedoch möglich, dass Teile des Medienstromes entlang der Außenwand des inneren Hohlkörpers in den Bereich zwischen innerem und äußerem Hohlkörper gelangen. Daher wird durch Dichtungselemente an dem vorstehend genannten Bereich ein Austritt effektiv verhindert.

Gemäß einer weiteren besonders bevorzugten Ausführungsform des erfindungsgemäßen Verschlusses sind der äußere und/oder der innere Hohlkörper und/oder das Unterteil rotationssymmetrisch ausgebildet. Vorzugsweise ist die Form rund. Somit erleichtern sich die Herstellung und die Handhabung. Insbesondere bei einer zylindrischen Ausführung vereinfacht sich die Konnektivität mit Schlauch- oder Rohrleitungen.

In einer weiteren vorteilhaften Ausgestaltung der Erfindung sind der äußere und/oder der innere Hohlkörper und/oder das Unterteil polygonal ausgebildet.

Gemäß einer weiteren bevorzugten Ausführungsform der Erfindung sind die Arretierungseinrichtungen umlaufende Dichtlippen oder Rippen. Solche Arretierungseinrichtungen gewährleisten eine wirkungsvolle Barriere gegenüber einem Abziehen des Unterteiles von dem Oberteil. Zu einer vereinfachten Ineinanderführung von Oberteil und Unterteil und zur gleichzeitigen Arretierung des Unterteilhohlkörpers innerhalb des Innen- und Außenzylinders des Oberteils können beispielsweise Dichtlippen dienen. Derartige Dichtlippen ermöglichen durch einen einseitig abgeschrägten Vorsprung ein einfaches Ineinanderstecken und verhindern ein späteres Herausziehen über diese Stelle hinaus.

Nach einer weiteren bevorzugten Ausführungsform der Erfindung ist die mindestens eine Halteeinrichtung ein Steg, der an der Innenwand des Unterteils angebracht ist und den Dorn hält.

Gemäß einer weiteren bevorzugten Ausführungsform der Erfindung ist der untere Bereich des Unterteils konisch ausgebildet. Hierbei ist der Ausdruck "untere Bereich" relativ zu dem Behälter zu verstehen, wenn der Verschluss an einem solchen angebracht ist. Durch den konischen Bereich können Schlauchleitungen sicher, fest und schnell angebunden werden. Auch andere Ausführungsformen des unteren Bereichs des Unterteils, wie Gewinde für Rohrleitungen, Klemmverbindungen etc. sind möglich. Der Durchmesser kann beliebig variiert werden.

Gemäß einer weiteren bevorzugten Ausführungsform der Erfindung ist der Verschluss beispielsweise durch Einschraubung, Einklebung, Einklemmung oder Einschweißung mit dem Behälter verbindbar. Der obere Teil des Oberteils ist bevorzugt als von der Öffnung durchbrochene Scheibe ausgebildet, welche mit dem Behälter durch vorstehend genannte Verbindungsmöglichkeiten verbindbar ist.

Gemäß einer weiteren, besonders bevorzugten Ausführungsform der Erfindung ist der Behälter ein Reaktor zum Einweggebrauch und aus flexiblem Kunststoff ausgebildet. Insbesondere bei Einwegprodukten ist es nötig, kostengünstige, aber dennoch sichere und einfach zu handhabende Verschlüsse zu schaffen, was durch die Erfindung erreicht wird.

Gemäß einer weiteren bevorzugten Ausführungsform des erfindungsgemäßen Verschlusses ist durch das Oberteil ein Schaft einer Mischeinrichtung des Behälters oder eine Begasungseinrichtung aufnehmbar. Durch die erfindungsgemäße Ausführung als nicht in den Behälterinnenraum hineinragender Verschluss kann beispielsweise ein Rührer oder eine Begasungseinrichtung ebenso wie der Verschluss selbst mittig angebracht werden, da Rührer oder Begasungseinrichtungen einer gleichmäßigen Verteilung ihres produzierten Prozessanteils (Strömung, Gasblasen) bedürfen. Ein Rührer wird ebenfalls bevorzugt beidseitig angebracht, da hierdurch eine besonders hohe Stabilität erreichbar ist. Eine Aufnahmevorrichtung kann beispielsweise an dem Verschluss angebracht oder in diesem integriert sein.

Gemäß einer weiteren bevorzugten Ausführungsform der Erfindung ist der Verschluss durch eine Überwurfmutter, einen Bajonett-Verschluss, eine Torsionsschnappverbindung oder eine Hülse mit Sollbruchstelle gegen eine unbeabsichtigte Öffnung zusätzlich gesichert. Weiterhin können diese Vorrichtungen eine Indikatorfunktion erfüllen, beispielsweise im Zusammenwirken mit RFID - Einrichtungen oder Computerprogrammprodukten, welche die unbeabsichtigte Öffnung des Verschlusses registrieren, speichern und einem Nutzer auslesbar zur Verfügung stellen. Ein weiteres Beispiel ist eine Hülse mit Sollbruchstellen. Die Hülse ist über Kragen an einer Seite mit dem Oberteil und an der anderen Seite mit dem Unterteil verbunden und hält diese ineinander. Durch Drehen der Kragen gegeneinander oder des Unterteils gegenüber dem Oberteil bricht die Hülse an Sollbruchstellen auf und der Verschluss kann geöffnet werden. Ein Bruch an der Hülse kann nun als Indikator genutzt werden, welcher die Benutzung des Abflusses anzeigt und somit zu mehr Prozesssicherheit und -kontrolle führt.

Gemäß einer weiteren bevorzugten Ausführungsform der Erfindung weisen das Oberteil und das Unterteil jeweils ein Schraubgewinde auf und das Unterteil ist in das Oberteil schraubbar. Somit können beispielsweise auch kleine Mengen des Mediums kontrolliert herausgelassen werden.

Die Figuren 1a bis 13 zeigen unterschiedliche Ausführungsformen des Verschlusses für einen Behälter.

In den Zeichnungen zeigen:
- Figur 1a: einen Querschnitt durch einen Verschluss,
- Figur 1b: eine perspektivische Ansicht eines Verschlusses,
- Figur 2a: eine Schnittansicht durch ein Ausführungsbeispiel des Verschlusses für einen Behälter im geöffnetem Zustand,
- Figur 2b: eine Schnittansicht durch ein Ausführungsbeispiel des Verschlusses für einen Behälter im geschlossenen Zustand,
- Figur 3a: eine Schnittansicht durch ein Ausführungsbeispiel des Unterteils,
- Figur 3b: eine Draufsicht auf eine Ausführungsform des Unterteils längs der Schnittlinie A-A der Figur 3a,
- Figur 4: eine Detailansicht eines Oberteils mit Dichtringen,
- Figur 5: eine Detailansicht eines Oberteils mit Dichtlippen,
- Figur 6: eine perspektivische Ansicht eines Verschlusses für einen Behälter mit einer Hülse mit Sollbruchstelle,
- Figur 7: eine perspektivische Ansicht eines Verschlusses für einen Behälter mit Sicherung durch eine Überwurfmutter,
- Figur 8: eine perspektivische Ansicht eines Ausführungsbeispieles des Verschlusses für einen Behälter mit Aufnahme für die Lagerung einer Rührerstange inklusive Durchgangsbohrungen,
- Figur 9: einen Querschnitt durch einen Verschluss für einen Behälter nach Figur 8 mit Aufnahme für die Lagerung einer Rührerstange inklusive Rührerstange und Lager,
- Figur 10: eine perspektivische Ansicht eines Verschlusses für einen Behälter als Einschraubversion,
- Figur 11: eine perspektivische Ansicht eines Verschlusses für einen Behälter als Anschweißversion,
- Figur 12: einen Querschnitt durch einen Verschluss für einen Behälter mit Schraubgewinde und
- Figur 13: einen Querschnitt durch einen Verschluss an einem Behälter mit einer Leitung an dem Schlauchanschluss.

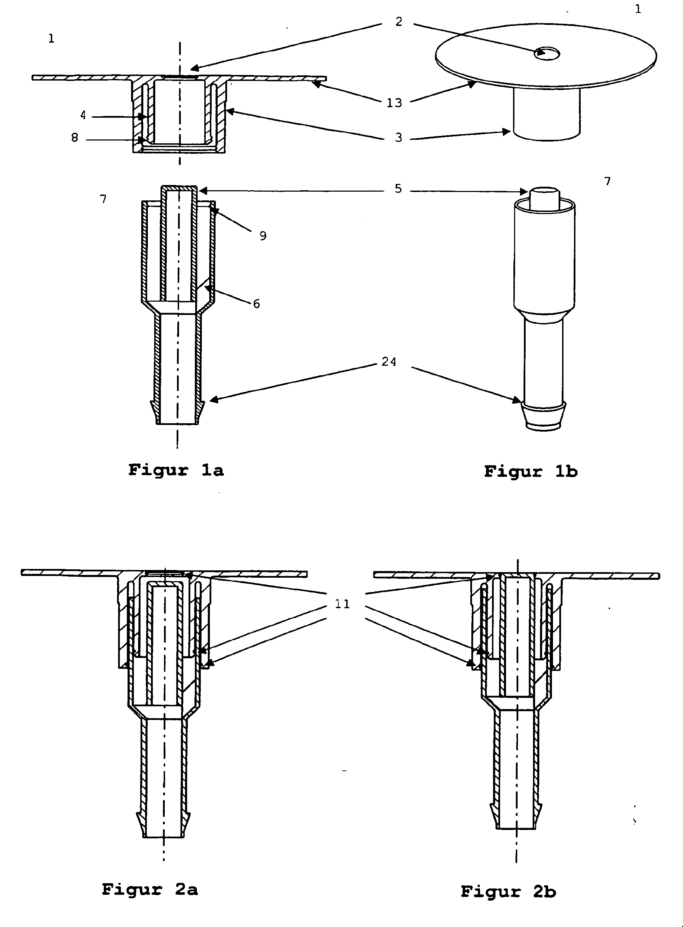

Gemäß Figur 1a bis Figur 2b besteht der Verschluss für einen Behälter aus einem mit dem Behälter verbindbaren Oberteil **1** und einem Unterteil **7.** Das Oberteil besitzt eine Öffnung **2** und weist einen äußeren Hohlkörper **3** und einen zu diesem koaxial angeordneten inneren Hohlkörper **4** auf. Der innere Hohlkörper **3** und der äußere Hohlkörper **4** bilden miteinander einen Zwischenraum aus. Ein Dorn **5** ist zum Verschließen der Öffnung **2** durch mindestens eine Halteeinrichtung **6** koaxial in einem hohlkörperförmigen Unterteil **7** aufgenommen, wobei der obere Teil des Unterteils **7** zum Öffnen der Öffnung **2** in dem Zwischenraum von dem Behälter weg längsverschiebbar ist. Die Längsverschiebbarkeit des Unterteils **7** ist durch Arretierungseinrichtungen **8, 9** begrenzt.

Figur 1a zeigt einen Querschnitt durch einen Verschluss für einen Behälter, wobei das Oberteil **1** und das Unterteil **7** voneinander getrennt dargestellt sind. Der selbe ist in Figur 1b perspektivisch dargestellt sowie als Querschnitt in Figur 2a im geöffneten Zustand und in Figur 2b im geschlossenen Zustand. Hierbei sind mögliche Dichtungselemente **11** gekennzeichnet.

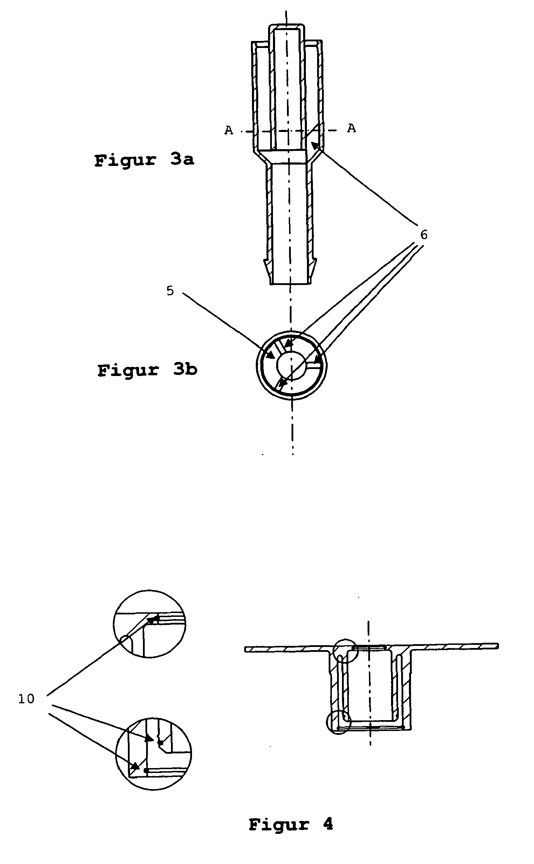

Figur 3b zeigt eine Draufsicht auf das Unterteil **7** entlang der Schnittlinie A-A der Figur 3a. In diesem Ausführungsbeispiel wurden drei Dornhalter **6** zur Befestigung des Dorns **5** angebracht. Dies ergibt bei ausreichendem Platz für das fließende Medium genügend Stabilität des Dorns **5.** Eine andere Anordnung, Art und Anzahl der Halteeinrichtung(en) **6** ist ebenso möglich.

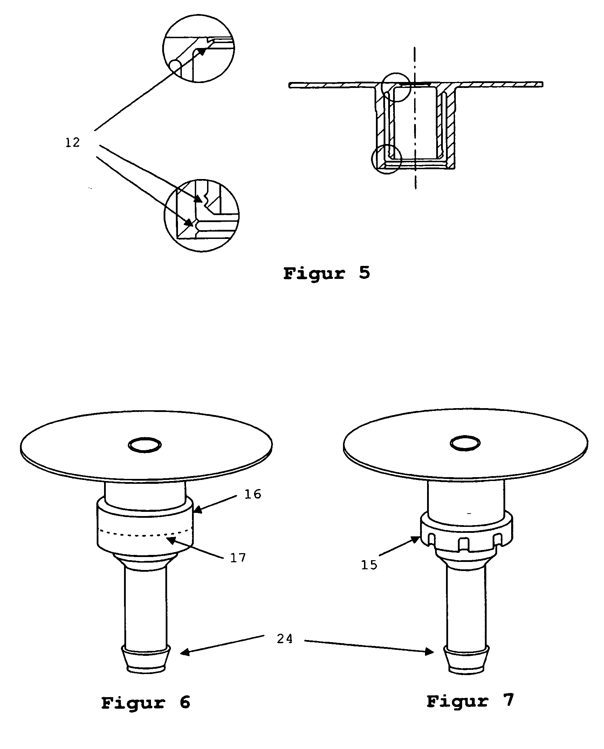

Figur 4 und Figur 5 sind Detailansichten je eines Beispieles mit Dichtringen **10** (Figur 4) bzw. Dichtlippen **12** (Figur 5).

Figur 6 und Figur 7 zeigen perspektivisch Ausführungen des Verschlusses für einen Behälter mit Sicherung durch eine Überwurfmutter **15** (Figur 7) oder durch eine Hülse mit Sollbruchstelle (n) **17** (Figur 6). Die Hülse **16,** wie in Figur 6 dargestellt, ist beispielsweise mit dem oberen Bereich an dem Oberteil **1** und mit dem unteren Bereich am Unterteil **7** befestigt. Diese Befestigung kann beispielsweise über Kragen erfolgen. Das Vorhandensein einer oder mehrerer Sollbruchstelle(n) **17** erleichtert einerseits die Öffnung des Verschlusses und kann gleichzeitig als Indikator dienen. Somit kann eine Vorbenutzung und versehentliches Öffnen erkannt werden und die Sterilität gewahrt werden. Zusätzlich hält eine Hülse **16** das Oberteil **1** und das Unterteil **7** sicher und effektiv zusammen. Mit Hilfe einer Überwurfmutter **15,** wie in Figur 7 dargestellt, kann dieser Zusammenhalt ebenso gewährleistet werden. Gleichzeitig kann eine derartige Ausführung auch als Öffnungs- bzw. Schließmechanismus dienen, wenn beispielsweise die Überwurfmutter **15** an ihrem unteren Bereich mit dem Unterteil **7** fest verbunden ist und über ihr, je nach Ausführungsform, Innen- bzw. Außengewinde um das entsprechende Gegengewinde am Oberteil ein- bzw. herausdrehbar ist. Ein ebenfalls drehbar gelagerter Schlauchanschluss **24** oder eine gleichartige Vorrichtung kann zusätzlich einer Verdrehung der Leitung **25** entgegenwirken.

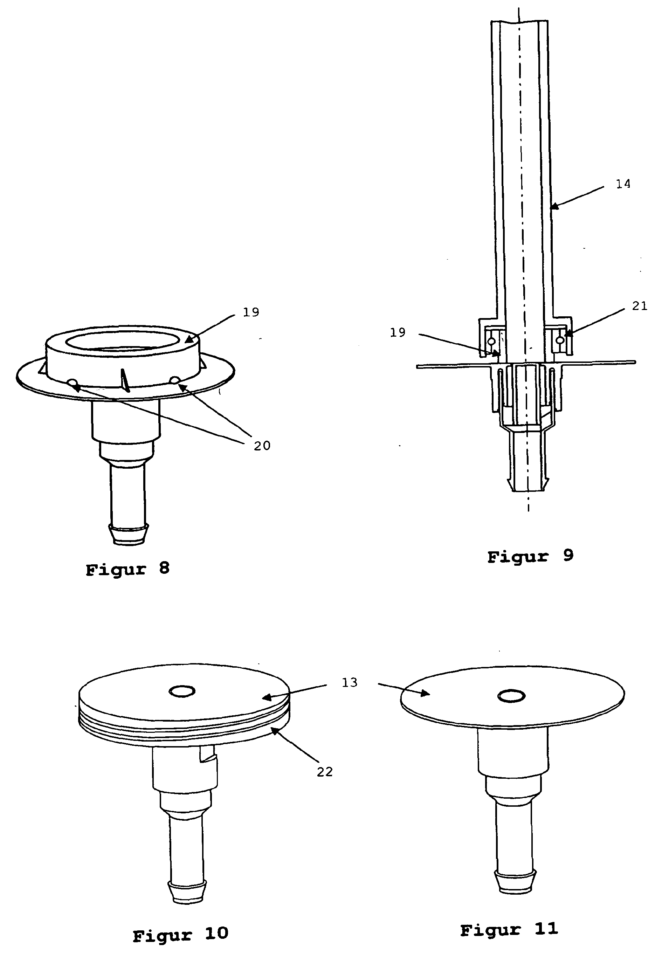

Das Ausführungsbeispiel der Figur 8 zeigt perspektivisch einen Verschluss für einen Behälter mit einer mit dem Oberteil **1** verbindbaren Aufnahme für die Lagerung eines Schaftes **14** inklusive Durchgangsbohrungen **20.** Für die Lagerungsaufnahme **19** sind zum Beispiel jegliche in Bioreaktoren verwendete Geräteteile, wie beispielsweise eine Begasungseinrichtung oder ein Rührer, denkbar. Um weiterhin einen Durchgang für das Medium zwischen Behälterinnenraum und Verschluss zu gewährleisten, sind beispielsweise Durchgangsbohrungen **20** an der Lagerungsaufnahme **19** oder anderweitige Durchbrechungen möglich. Die Lagerungsaufnahme **19** kann als fester Bestandteil des Oberteils **1** hergestellt werden, zum Beispiel mittels Spritzgießverfahren in einem Stück, oder als separate Vorrichtung im Nachhinein an das Oberteil **1** angebracht werden, beispielsweise durch Verkleben, Verschweißen, Einklemmen oder Einschrauben. Es ist auch möglich, die Lagerungsaufnahme **19** direkt mit dem Behälter zu verbinden. Ein Ausführungsbeispiel mit dargestelltem Schaft **14** in der Lagerungsaufnahme **19** sitzend ist in Figur 9 im Querschnitt dargestellt. Hier ist ein an der Lagerungsaufnahme **19** sitzendes Lager **21** dargestellt, welches einen Rührerschaft drehbar lagert. Der Schaft **14** kann, wie bereits erwähnt, auch einen Teil eines anderen Gerätes darstellen oder zur Stabilisierung des Behälters dienen.

Figur 10 und Figur 11 stellen weitere Beispiele des Oberteils **1** eines Verschlusses für einen Behälter dar. Der Verschluss kann entsprechend den Anforderungen, Bedürfnissen oder aus Gründen der Materialgleichheit aus beliebigen Materialien gefertigt werden. So kann beispielsweise die Scheibe **13** als ein Einschraubteil ausgeführt sein und mit einem Gewinde **22,** wie es häufig für Metallbehälter vorgesehen ist, ausgestattet sein, wie die perspektivische Ansicht der Figur 10 darstellt. Eine solche Ausführung aus Kunststoff oder anderen Materialien ist ebenso denkbar. Figur 11 zeigt perspektivisch ein Ausführungsbeispiel, welches als Anschweißteil oder Anklebteil das Anschweißen oder Ankleben an eine Behälterinnen- oder Behälteraußenwandung gestattet. Bei Einsatz in Kunststoffbehältern beispielsweise weist das Oberteil **1** bevorzugt eine Scheibe **13** auf, welche mit der Wandung des Kunststoffcontainers verschweißt ist.

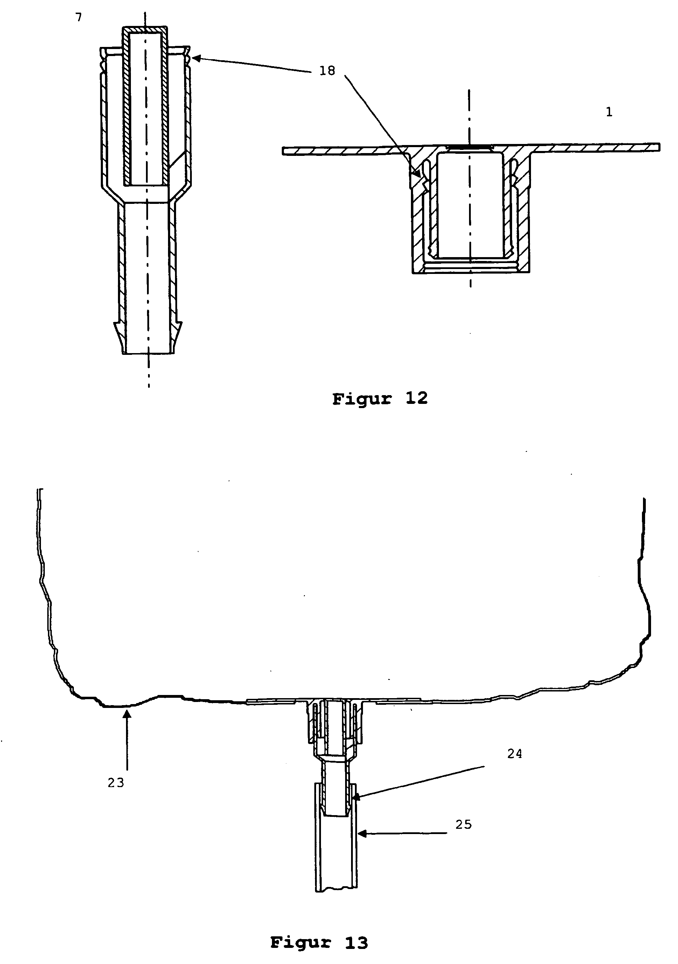

Die in Figur 12 im Querschnitt dargestellte Form weist ein Schraubgewinde **18** für den Verschluss auf. Das Oberteil **1** und das Unterteil **7** sind jeweils mit einem Gewinde versehen, so dass das Unterteil **7** in das Oberteil **1** eingeschraubt werden kann. Das Öffnen bzw. das Schließen des Verschlusses geschieht durch Drehung des Unterteils **7** gegen das Oberteil **1**. Hierfür kann, wie in Figur 12 dargestellt, das Unterteils **7** an der Außenwand mit einem Innengewinde und das Oberteil **1** an der Innenwand des äußeren Hohlkörpers **3** mit einem Außengewinde versehen sein. Die Schraubgewinde **18** können auch an anderen Stellen angebracht bzw. integriert sein und auch in ihrer Ausführungsart (Innen- bzw. Außengewinde) miteinander beliebig vertauscht sein, beispielsweise indem an der Innenwand des Unterteils **7** ein Außengewinde und an der Außenwand des inneren Hohlkörpers **4** ein Innengewinde angebracht oder integriert ist. Es ist auch möglich, durch Bohrung zwischen dem äußeren Hohlkörper **3** und dem inneren Hohlkörper **4** ein Muttergewinde zu schaffen, in welches das Unterteil **7,** als Bolzengewinde ausgeführt, eingedreht werden kann. Das Schraubgewinde **18** kann beispielsweise als Metrisches-ISO-Gewinde, Whitworthgewinde, Rohrgewinde, Trapezgewinde, Rundgewinde oder Sägengewinde ausgeführt sein. Durch die Verwendung eines Schraubgewindes **18** lässt sich ein Medium kontrolliert zu- und abführen. Wird beispielsweise ein Feingewinde eingesetzt, erlaubt der geringe Vorschub pro Umdrehung präzise Einstellungen. Durch die Ausführung mit Gewinde wird allgemein ein hohes Maß an Stabilität, Festigkeit, Flexibilität und Sicherheit gegeben.

Figur 13 zeigt einen Querschnitt durch ein Beispiel des Verschlusses für einen Behälter, wobei das Oberteil **1** an einem Einwegbehälter **23** befestigt dargestellt ist. Aus Gründen der Übersichtlichkeit ist nur ein Bereich des Einwegbehälters **23** ohne zusätzliche Elemente abgebildet. Das Unterteil **7** ist seinerseits mit dem Oberteil **1** zusammengefügt und mit einer Leitung **25** dargestellt, die an den Schlauchanschluss **24** des Unterteils **7** angebracht ist. Das Unterteil **7** kann im allgemeinen an jeden beliebigen Schlauch- oder Rohrdurchmesser angepasst sein.

### Bezugszeichenliste:

- 1: Oberteil
- 2: Öffnung
- 3: Äußerer Hohlkörper
- 4: Innerer Hohlkörper
- 5: Dorn
- 6: Halteeinrichtung(en)
- 7: Unterteil
- 8: Arretierungseinrichtung
- 9: Arretierungseinrichtung
- 10: Dichtring
- 11: Dichtungselement(e)
- 12: Dichtlippen
- 13: Scheibe
- 14: Schaft
- 15: Überwurfmutter
- 16: Hülse
- 17: Sollbruchstelle(n)
- 18: Schraubgewinde
- 19: Lagerungsaufnahme
- 20: Durchgangsbohrungen
- 21: Lager
- 22: Gewinde
- 23: Einwegbehälter
- 24: Schlauchanschluss
- 25: Leitung

## Patentansprüche

1. Verschluss für einen Behälter, umfassend:
- ein mit dem Behälter verbindbares Oberteil (1) mit einer Öffnung (2), das einen äußeren Hohlkörper (3) und einen zu diesem koaxial angeordneten inneren Hohlkörper (4) aufweist, der mit dem äußeren Hohlkörper (3) einen Zwischenraum ausbildet, wobei der obere Teil des Oberteils (1) als von der Öffnung (2) durchbrochene Scheibe (13) ausgebildet ist, die mit dem Behälter verbindbar ist, und eine Lagerungsaufnahme (19) zur Aufnahme von in dem Behälter zu verwendenden Geräteteilen umfasst,
- ein hohlkörperförmiges Unterteil (7),
- einen Dorn (5) zum Verschließen der Öffnung (2), der durch mindestens eine Halteeinrichtung (6) koaxial in dem hohlkörperförmigen Unterteil (7) aufgenommen ist,
wobei der obere Teil des Unterteils (7) zum Öffnen der Öffnung (2) in dem Zwischenraum von dem Behälter weg langsverschiebbar ist und wobei die Längsverschiebbarkeit des Unterteils (7) durch Arretierungseinrichtungen (8, 9) begrenzt ist.

2. Verschluss nach Anspruch 1, wobei die Arretierungseinrichtungen (8, 9) an der Innenwand des Unterteils (7) und an der Außenwand des inneren Hohlkörpers (4) angeordnet sind.

3. Verschluss nach einem der vorhergehenden Ansprüche, wobei im verschlossenen Zustand das obere Ende des Dorns (5) bündig mit dem Oberteil (1) abschliesst.

4. Verschluss nach einem der vorhergehenden Ansprüche, wobei der Dorn (5) durch einen Dichtring (10) oder Dichtlippen (12) gegenüber der Öffnung (2) des Oberteils (1) abgedichtet ist.

5. Verschluss nach einem der vorhergehenden Ansprüche, wobei die Innenwand des äusseren Hohlkörpers (3) und die Aussenwand des inneren Hohlkörpers (4) je mindestens ein Dichtungselement (11) aufweisen.

6. Verschluss nach einem der vorhergehenden Ansprüche, wobei die Arretierungseinrichtungen (8, 9) umlaufende Dichtlippen (12) oder Rippen sind.

7. Verschluss nach einem der vorhergehenden Ansprüche, wobei die mindestens eine Halteeinrichtung (6) ein Steg ist, der an der Innenwand des Unterteils (7) angebracht ist und den Dorn (5) hält.

8. Verschluss nach einem der vorhergehenden Ansprüche, wobei der untere Bereich des Unterteils (7) konisch ausgebildet ist.

9. Verschluss nach einem der vorhergehenden Ansprüche, wobei der Verschluss durch Einschraubung, Einklebung, Einklemmung oder Einschweissung mit dem Behälter verbindbar ist.

10. Verschluss nach einem der vorhergehenden Ansprüche, wobei die Lagerungsaufnahme (19) ein Lager (21) zum drehbaren Lagern von Geräteteilen umfasst.

11. Verschluss nach einem der vorhergehenden Ansprüche, wobei an die Lagerungsaufnahme (19) des Oberteils (1) ein Schaft (14) koppelbar ist.

12. Verschluss nach einem der vorhergehenden Ansprüche, wobei an die Lagerungsaufnahme (19) des Oberteils (1) eine Begasungseinrichtung koppelbar ist.

13. Verschluss nach einem der vorhergehenden Ansprüche, wobei der Verschluss durch eine Überwurfmutter (15), einen Bajonett-Verschluss, eine Torsionsschnappverbindung oder eine Hülse (16) mit Sollbruchstelle (n) (17) gegen eine unbeabsichtigte Öffnung gesichert ist.

14. Verschluss nach einem der vorhergehenden Ansprüche, wobei das Oberteil (1) und das Unterteil (7) jeweils ein Schraubgewinde (18) aufweisen und das Unterteil (7) in das Oberteil (1) schraubbar ist.

## Claims

1. A closure for a container, comprising:
- a top part (1) connectable with the container with an opening (2), which has an outer hollow body (3) and an inner hollow body (4) arranged coaxially to it, which forms an intermediate space with the outer hollow body (3), wherein the upper part of the top part (1) is designed as a disk (13) penetrated by the opening (2), which is connectable with the container, and a mounting receiver (19) for receiving device parts to be used in the container, comprising,
- a hollow-body-shaped bottom part (7),
- a mandrel (5) for closing the opening (2), which is received by at least one holding device (6) coaxially in the hollow-body-shaped bottom part (7),
wherein the upper part of the top part (7) is longitudinally shiftable away from the container for opening the opening (2) in the intermediate space and wherein the longitudinal shiftability of the bottom part (7) is restricted by locking devices (8, 9).

2. The closure according to claim 1, wherein the locking devices (8, 9) are arranged on the inner wall of the bottom part (7) and on the outer wall of the inner hollow body (4).

3. The closure according to one of the previous claims, wherein the upper end of the mandrel (5) in the closed state is flush with the top part (1).

4. The closure according to one of the previous claims, wherein the mandrel (5) is sealed with respect to the opening (2) of the top part (1) by a sealing ring (10) or sealing lips (12).

5. The closure according to one of the previous claims, wherein the inner wall of the outer hollow body (3) and the outer wall of the inner hollow body (4) each have at least one sealing element (11).

6. The closure according to one of the previous claims, wherein the locking devices (8, 9) are circumferential sealing lips (12) or ribs.

7. The closure according to one of the previous claims, wherein the at least one holding device (6) is a bar, which is attached to the inner wall of the bottom part (7) and holds the mandrel (5).

8. The closure according to one of the previous claims, wherein the lower area of the bottom part (7) is designed conically.

9. The closure according to one of the previous claims, wherein the closure is connectable with the container through screwing in, gluing in, clamping in or welding in.

10. The closure according to one of the previous claims, wherein the mounting receiver (19) comprises a mount (21) for the rotatable mounting of device parts.

11. The closure according to one of the previous claims, wherein a shaft (14) can be coupled to the mounting receiver (19) of the top part (1).

12. The closure according to one of the previous claims, wherein a fumigation device can be coupled to the mounting receiver (19) of the top part (1).

13. The closure according to one of the previous claims, wherein the closure is secured from accidental opening by a union nut (15), a bayonet closure, a torsion snap connection or a shell (16) with predetermined breaking point (n) (17).

14. The closure according to one of the previous claims, wherein the top part (1) and the bottom part (7) each have a screw thread (18) and the bottom part (7) can be screwed into the top part (1).

## Revendications

1. Bouchon pour un contenant, comprenant : une partie supérieure (1) avec un orifice (2), la partie supérieure pouvant être raccordée au contenant et présentant un corps creux extérieur (3) et un corps creux intérieur (4) disposé coaxialement par rapport à ce dernier et formant avec le corps creux extérieur (3) un espace intermédiaire, dans lequel le haut de la partie supérieure (1) est formé comme disque ajouré (13) par l'orifice (2), le disque pouvant être raccordé au contenant, et un logement-support (19) pour le logement de pièces d'appareils à utiliser dans le contenant,
une partie inférieure (7) en forme de corps creux,
une broche (5) pour la fermeture de l'orifice (2) logée par au moins un dispositif de retenue (6) coaxialement dans la partie inférieure (7) en forme de corps creux,
dans lequel le haut de la partie inférieure (7) peut être décalé longitudinalement dans l'espace intermédiaire, en s'éloignant du contenant, pour ouvrir l'orifice (2), et dans lequel la mobilité longitudinale de la partie inférieure (7) est limitée par des dispositifs d'arrêt (8, 9).

2. Bouchon selon la revendication 1, dans lequel les dispositifs d'arrêt (8, 9) sont disposés sur la paroi intérieure de la partie inférieure (7) et sur la paroi extérieure du corps creux intérieur (4).

3. Bouchon selon l'une quelconque des revendications précédentes, dans lequel, à l'état fermé, l'extrémité supérieure de la broche (5) est alignée avec la partie supérieure (1).

4. Bouchon selon l'une quelconque des revendications précédentes, dans lequel la broche (5) est étanchée par une bague d'étanchéité (10) ou des lèvres d'étanchéité (12) en face de l'orifice (2) de la partie supérieure (1).

5. Bouchon selon l'une quelconque des revendications précédentes, dans lequel la paroi intérieure du corps creux extérieur (3) et la paroi extérieure du corps creux intérieur (4) présentent chacune au moins un joint d'étanchéité (11).

6. Bouchon selon l'une quelconque des revendications précédentes, dans lequel les dispositifs d'arrêt (8, 9) sont des lèvres d'étanchéité (12) ou des nervures circonférentielles.

7. Bouchon selon l'une quelconque des revendications précédentes, dans lequel le au moins un dispositif de retenue (6) est une arête fixée à la paroi intérieure de la partie inférieure (7) et retenant la broche (5).

8. Bouchon selon l'une quelconque des revendications précédentes, dans lequel la zone inférieure de la partie inférieure (7) a une forme conique.

9. Bouchon selon l'une quelconque des revendications précédentes, dans lequel le bouchon peut être raccordé au contenant par vissage, collage, serrage ou soudage.

10. Bouchon selon l'une quelconque des revendications précédentes, dans lequel le logement-support (19) comprend un palier (21) pour le support rotatif de pièces d'appareils.

11. Bouchon selon l'une quelconque des revendications précédentes, dans lequel le logement-support (19) de la partie supérieure (1) peut être couplé à une tige (14).

12. Bouchon selon l'une quelconque des revendications précédentes, dans lequel le logement-support (19) de la partie supérieure (1) peut être couplé à un dispositif de gazage.

13. Bouchon selon l'une quelconque des revendications précédentes, dans lequel le bouchon est protégé contre toute ouverture involontaire par un écrouraccord (15), un joint à baïonnette, un moyen de connexion par encliquetage à torsion ou une douille (16) avec point(s) de rupture (17).

14. Bouchon selon l'une quelconque des revendications précédentes, dans lequel la partie supérieure (1) et la partie inférieure (7) présentent respectivement un filetage (18) et la partie inférieure (7) peut être vissée dans la partie supérieure (1).
